# EUROPÄISCHE PATENTANMELDUNG

(11) **EP 2 883 527 A1**
(43) Veröffentlichungstag der Anmeldung: **17.06.2015**
(21) Anmeldenummer: 13196496.7
(22) Anmeldetag: 10.12.2013
(51) Int. Cl.: A61K 6/00

(54) **Dentalmaterial**

(71) Anmelder: Universität Ulm, 89081 Ulm (DE)
(72) Erfinder: Luthardt, Ralph G., 89075 Ulm (DE); Martin, Thomas, 89075 Ulm (DE); Rudolph, Heike, 86495 Eurasburg (DE)
(74) Vertreter: Pfenning, Meinig & Partner GbR

(57) **Zusammenfassung**

Die Erfindung betrifft Dentalmaterialien zum Management des parodontalen und periimplantären Weichgewebes bei der zahnärztlichen Behandlung. Das erfindungsgemäße Dentalmaterial zeichnet sich dadurch aus, dass es ein selbsthärtendes, mechanisch bearbeitbares Material ist mit einer Shore A-Härte > 20. Die Erfindung betrifft weiterhin ein Verfahren zum Management des paradontalen und periimplantären Weichgewebes mit einem Material wie vorstehend beschrieben und dessen Verwendung.

## Beschreibung

Die Erfindung betrifft Dentalmaterialien zum Management des parodontalen und periimplantären Weichgewebes bei der zahnärztlichen Behandlung. Das erfindungsgemäße Dentalmaterial zeichnet sich dadurch aus, dass es ein selbsthärtendes, mechanisch bearbeitbares Material ist mit einer Shore A-Härte > 20. Die Erfindung betrifft weiterhin ein Verfahren zum Management des paradontalen und periimplantären Weichgewebes mit einem Material wie vorstehend beschrieben und dessen Verwendung.

Die Präparation eines Zahnes für die spätere Versorgung wie einer Restauration, erfordert die klare Darstellung der Präparationsränder und bei vielen Restaurationsarten auch ein Schutz vor Feuchtigkeit oder Blut um eine ausreichende Befestigung zu ermöglichen. Dieser Schutz wird in der Regel durch Watterollen, Kofferdam, Fadentechnik bzw. Doppelfadentechnik und Retraktionsmaterialien gewährleistet.

Retraktionsmaterialien sind in der Regel pastöse Einlagematerialien, die vor einer Abformung (digital oder klassisch per bekannter Abformmaterialien zum Beispiel Silikone) aus dem Sulkus/periimplantären Gewebeentfernt, meist ausgewaschen werden. Sie sollen den Sulkus öffnen bzw. die/die periimplantären Gewebe stabilisieren, um ein Einfließen der Abformmasse zu gewährleisten und eine Kontrolle von Blut und Feuchtigkeit zu ermöglichen, was in der Regel durch Zusätze von Astringentien erfolgt. Derartige Retraktionspasten sind z.B. in der WO 2009/092568 A1 beschrieben.

Nachteilig bei diesen Pasten ist:
- Paste wird vor der Abformung ausgewaschen, wodurch sich der Sulkus wieder schließen kann
- Keine Digitalisierbarkeit
- Keine Bearbeitbarkeit
- Nur lokale Isolationsfunktion bzw. Absorptionsfunktion
- Geringe bis keine Schutzfunktion für marginale Gingiva während der Präparation

Auch die bisher weit verbreitete Fadentechnik oder auch Doppelfadentechnik weist Nachteile auf.

Nachteilig ist dabei, dass die Gingiva stranguliert wird und dadurch eine Unterversorgung des Gewebes verursachen kann.

Weitere Nachteile der Fadentechnik sind die folgenden:
- Sehr zeitaufwändig
- Schwer in die Präparation eines Zahnes integrieren
- Geringe bis keine Schutzfunktion für marginale Gingiva während der Präparation
- Nur lokale Isolationsfunktion
- Keine Bearbeitbarkeit
- Eingeschränkt für intraorale Digitalisierung zu gebrauchen

Nachteilig bei allen vorstehend beschriebenen Methoden, d.h. sowohl bei den Retraktionsmaterialien wie auch bei der Fadentechnik ist aber, dass durch diese Methoden ein großer Zeitaufwand bei der Anwendung entsteht und auch eine schwere Handhabbarkeit vorhanden ist. Nachteilig ist auch, dass die Retraktionsmaterialien vor der Abformung wieder entfernt werden müssen, wodurch es auch vorkommen kann, dass noch Reste im Sulkus verbleiben.

Ausgehend hiervon ist die Aufgabe der vorliegenden Erfindung es deshalb, ein Dentalmaterial vorzuschlagen, das in seiner Handhabung sehr einfach ist und das insbesondere über die gesamte Dauer der zahnärztlichen Behandlung Schutz vor Feuchtigkeit, Blutung und Sulkusfluid gewährleistet.

Die Aufgabe wird durch die kennzeichnenden Merkmale des Patentanspruchs 1 in Bezug auf das Material und bezüglich des Verfahrens durch die Merkmale des Anspruches 8 gelöst. Die Unteransprüche zeigen vorteilhafte Weiterbildungen auf.

Erfindungsgemäß wird somit ein Dentalmaterial vorgeschlagen, das selbsthärtend ist und eine Shore A-Härte des ausgehärteten Materials aufweist, die größer 20 ist. Bei dem Dentalmaterial der Erfindung ist besonders hervorzuheben, dass dadurch, dass es bearbeitbar ist, das Material während der Abformung/Digitalisierung nicht entfernt werden muss. Das Material kann somit temporärin die zahnärztliche Restauration mit einbezogen werden, d.h. das Material selbst wird mit bearbeitet und erst anschließend wieder entfernt. Das Material nach der Erfindung unterscheidet sich somit dadurch von konventionellen Retraktionsmaterialien, dass es schabbar, fräsbar sowie digitalisierbar ist und damit in die Bearbeitung mit einbezogen werden kann. Das Material nach der Erfindung kann somit in den Bearbeitungsvorgang der Zahnhartsubstanz oder andere intraorale Gewebe integriert werden. Durch die abdichtenden Eigenschaften des Materials ermöglicht die Erfindung außerdem ein Abfließen unterhalb der Isolationsgrenze. Die Erfindung ermöglicht dadurch eine exakte Abbildung der Präparationsgrenzen
- durch Kontrastierung
- durch ihre exakte Abbildungsgenauigkeit, und
- durch ihre Schutzfunktion vor Kontamination.

Das Dentalmaterial nach der Erfindung weist dabei nach Aushärtung eine Shore A-Härte von mindestens 20, bevorzugt bis 98 und eine Shore D-Härte von mindestens 20 bis 65 auf.

Die Shore-Härte wird analog in den Normen DIN 53505 und DIN 7868 bestimmt. Die Eindringtiefe eines Gewichtsbelasteten Metallstiftes in das zu prüfende Material ist ein Maß für die Shore-Härte, die auf einer Skala von 0 Shore (2,5 Millimeter Eindringtiefe) bis 100 Shore (0 Millimeter Eindringtiefe) gemessen wird. Eine hohe Zahl bedeutet eine große Härte. Bei der Bestimmung der Shore-Härte spielt die Temperatur eine höhere Rolle als bei der Härtebestimmung metallischer Werkstoffe. Deshalb wird hier die Solltemperatur von 23 °C auf das Temperaturintervall von ± 2 K beschränkt. Die Materialdicke sollte mindestens 6 Millimeter betragen. Die Härte des Gummis wird durch die Vernetzung (schwach vernetzt = Weichgummi, stark vernetzt = Hartgummi) bestimmt. Aber auch der Gehalt an Füllstoffen ist für die Härte eines Gummiartikels ausschlaggebend.

Shore-A wird angegeben bei Weich-Elastomeren, nach Messung mit einer Nadel mit abgestumpfter Spitze. Die Stirnfläche des Kegelstumpfs hat einen Durchmesser von 0,79 Millimetern, der Öffnungswinkel beträgt 35°. Auflagegewicht: 1 kg, Haltezeit: 15 s. Handmessgeräte müssen meist sofort beim Aufdrücken auf die Probe abgelesen werden, der angezeigte Wert nimmt bei längerer Haltezeit ab.

Shore-D wird angegeben bei Zäh-Elastomeren nach Messung mit einer Nadel, die mit einem 30°-Winkel zuläuft und eine kugelförmige Spitze mit einem Radius von 0,1 Millimetern hat. Auflagegewicht: 5 kg, Haltezeit: 15 s Das Dentalmaterial nach der Erfindung ist dabei bevorzugt ein Ein- oder Zweikomponentensystem. In der Regel eine Paste mit einem Katalysator. Bei der Aushärtung durch Lichthärtung kann die Aufbewahrung in einer lichtgeschützten Kapsel erfolgen. Bei einer Zwei-Komponenten -Variante erfolgt die Aufbewahrung in einer handelsüblichen Kartusche mit oder ohne integrierterDosiereinrichtung mit getrennten Kammern und wird erst bei der Applikation vermischt.

Das Dentalmaterial ist dabei bevorzugt ausgewählt aus Silikonen, Polyethern, Kunststoffe auf Harz oder Methylacrylatbasis und/oder Komposites.

Nachfolgend sind Beispiele für die Materialien angegeben:
a) Silikone: Polyvinylsiloxan, Polymethylsiloxan, Polyvinylsiloxanether Zubereitung aus Silikonpolymeren und Füllstoffen mit (Platin-) Katalysator (im ppm- Bereich). Das Material kann dabei hydrophoben Charakter beibehalten oder hydrophilisiert werden.
b) Polyether deren Ausgangspunkt Copolymerisate aus Äthylenoxid Tetrahydrofuran sind
c) Polysulfide: auch Thiokole, Basispaste: Mekromolekulare Polysulfide mit endständigen und im Mittel einer weiteren seitständigen reaktionsfähigen SH-Gruppe (Merkaptane) sowie Füllstoffe (ZnO, CaSO4, TiO2: 10-55 %, je nach Fließfähigkeit), Katalysatorpaste: Bleidioxid (50-80 m%), bis 4 m% und ein geeignetes Öl (z.B. Paraffin).
d) Kunststoffe auf Harz oder Methylacrylatbasis (Polymere des Methylacrylsäuremethylesters) und/oder Kompositionsmassen (Hochschmelzende, feste Harze ca. 35m% und Weichmachern wie Wachse, Paraffine, Stearinsäure ca. 10 m% und Füll-und Farbstoffe ca. 55m%
   Alkalisch gelöste Acrylharze:
   Schellackseife; Schellack verseift mit Ammoniak (alternative Alkalia: Kalilauge oder Natronlauge)
e) Komposite: Mischung hochmolekularer, mehrfunktioneller Methacrylate und/oder Acrylate unter Zusatz von Füllstoffen wie Mikrofüller z.B. Siliziumoxid, Barium-oder Strontiumgläsern. Auch Zusätze von Yttrium oder Ytterbiumfluorid.

Die Dentalmaterialien können, wie im Stand der Technik bekannt, verschiedene Additive enthalten. Bevorzugt sind die folgenden Additive:
Füllstoffe:
   Füllstoffe, die in der Regel den Metalloxiden zuzuordnen sind. Dazu zählen unter anderem Titanoxid (TiO₂), Bariumoxid, Calciumcarbonat, Calciumsulfit, Siliziumoxid, Zinnoxid, Zirkonoxid oder Aluminium-Zirkoniumoxid-Dispersion oder andere, die zum Zwecke der Reflektion ohne Streustrahlung, ausgehend von einer Lichtquelle eines optischen Digitalisierungssystems, die Erfassung durch dieses Digitalisierungssystem ermöglichen, ohne dabei die Eigenschaft eines Volumenstreuers aufzuweisen. Der Füllstoffgehalt liegt bei größer 2% bezogen auf die Gesamtmenge der Paste.

Weitere geeignete Füllstoffe sind Lithopane, ungiftiges Pigment aus Baryt (Bariumsulfat) und Zinksulfid (ZnS), sowie Metalloxide: z.B. Aluminiumoxid (Al₂O₃) oder Zirkoniumdioxid (ZrO₂) oder Dispersion mit einem Anteil von bis zu 40% Al₂O₃.

Die Erfindung umfasst auch Mischungen der Füllstoffe. Auch können weitere Additive wie z.B. Absorber, z.B. Tusche, Ruß oder Eisensulfat oder fluoreszierende Stoffe zugemischt werden.

Die Partikelgrößen der Füllstoffe, liegen im Bereich von > 50 nm und < 100 µm.

### Superabsorptionssystem

Superabsorberpolymere abgeleitet von Acrylsäure oder Methacrylsäure mit Partikelgrößen zwischen 100 und 900 µm, wobei ein gewisser Anteil an wasserlöslichen Feststoffen beigefügt ist z.B. Polyacrylsäure. Superabsorberpartikel, die nach Kontakt mit Flüssigkeit und deren Aufnahme aufquellende Eigenschaft besitzen. Die Partikelgröße kann zwischen 40nm und 350µm liegen. Quellung und Expansion durch Absorption sorgt für eine zusätzliche mechanische Aufweitung des Sulkus. Die initiale Aufweitung des Sulkus erfolgt durch den reinen Applikationsdruck von 20-35 g.

### Hämostatika

Zusätze von Hämostatika, wie Aluminiumchlorid, Alaun, Kaliumalaun, Aluminiumsulfat etc. Dies sind im Allgemeinen wasserlösliche Aluminiumsalze, sowie Mangan- oder Wismuthsalze, Eisen-, oder Zinksalze oder Alkalihalogenide. Auch Zusätze wie Adrenalin, Epinephrin, Noradrenalin, Gerbsäuren oder andere Vasokonstriktoren und Adstringenzien wie Calciumsalze sind vorstellbar.

Weitere bevorzugte Additive sind Iniatiatoren, Farbstoffen, Adstringentien, Desinfektionsmitteln, Vasokonstrikoren, Lösungsmittel und Desensitizer wie z.B. Fluoride, Kalium- und Strotiumsalze, Calciumhydrogenphosphat oder Hydroxylapatit sowie Zusätze für die Thixotropie sowie Geschmacksstoffe.

Zusätze für die Thixotropie werden in einer Mischung von 1-15 Gewichtsprozent, bezogen auf die Paste, eines organischen Thixotropiermittels, z.B. Polyglykolether von Glyzeriden oder hydriertes Rizinusöl eingesetzt.

### Farbstoffe bieten zusätzlich den Vorteil, dass dadurch die Einstellbarkeit von

Kontrast, Reflexion und Absorption möglich ist. Für Materialien die mittels UV polymerisiert werden, sind in der Regel bifunktionelle Monomere (Vernetzer) notwendig, z.B Urethandimethacrylat UDMA; auch UV Absorber z.B. Derivate des Benzophenons; Photoinitiatoren z.B. Campherchinon.

Die Applikation des Materials kann durch handelsübliche Applikationspistolen mit wechselbaren Aufsätzen (Kombination von Mischwendel und intraoraler Applikationsdüse) erfolgen dazu auch Kapselsysteme mit integrierter Applikationsspitze, für dünne, sulkusgerechte Applikation oder größere Durchmesser für großflächigere Applikation, je nach klinischem Anwendungszweck und benötigter Fläche. Die Durchmesser der Einbringkanülen/Applikationsaufsätze variieren zwischen 0,2 und 5 mm.

Die Viskosität des Materials vor Aushärtung liegt bei mindestens 500 mPa.s bei 23° (niederviskös, Fließfähigkeit vergleichbar Öl), bevorzugt zwischen 10.000 und 100.000 mPa.s bei 23 °C, in Abhängigkeit vom Basismaterial auch bis zu 800.000 mPa.s bei 23 °C (hochviskös, vergleichbar knetbarem Putty-Abformmaterial).

Die Aushärtung des Materials erfolgt in einem Zeitraum zwischen 20s und 10 min. Während der Aushärtungsphase bei einer Verarbeitungstemperatur zwischen 15 und 40 Grad zeichnet sich das Material durch eine hohe Viskosität aus, um ein gutes Einfließen in den Sulkus zu ermöglichen.

Um die Verarbeitungszeit zu steuern können auch unterschiedliche Beschleuniger/Verzögerer beigemengt (Katalysatoren, Salze o.ä.) werden.

Das Material kann auch röntgenopak ausgestattet sein. Dadurch können Materialreste aufgefunden werden. Dies kann beispielsweise durch Zugabe von Titanoxid oder Barium oder andere für die Einstellung der Röntgenopaziät verfügbare Materialien erreicht werden.

Die Zusammensetzungen der erfindungsgemäßen Materialien sind so ausgewählt, dass sie die mechanische Aufweitung gewährleisten. Die Viskosität wird dabei durch den Füllstoffgehalt gewährleistet. Außerdem muss die Möglichkeit der Datenerfassung durch entweder Reflexion oder Absorption gegeben sein.

Die Abbindereaktion kann chemisch und/oder lichthärtend erfolgen. Auch eine physikalische Aushärtung ist von der Erfindung umfasst.

Das Material der Erfindung kann restlos aus dem Sulkus entfernt werden. Es wird meist in Bruchfragmenten entfernt. Dies ist bei den weichbleibenden Retraktionspasten nur sehr schwer möglich, da durch ein Auswaschen oder kombiniertes Auswaschen/Auspusten nicht gewährleistet werden kann, dass die Pasten vollständig aus dem Sulkus entfernt worden sind. Das vollständig aushärtende Material der Erfindung gewährleistet durch seine Stabilität und Abformtreue, dass eine gute visuelle Beurteilung von Bruchfragmenten möglich ist, und somit eventuell vorhandene Reste aufgesucht und entfernt werden können. Aufgrund der Restelastizität des Materials, ist ein Abreißen, auch dünner "Fließfahnen" ausgeschlossen sein.

In Abhängigkeit von der verwendeten Grundsubstanz liegt der Gewichtsanteil an Inhaltsstoffen bezogen auf die Gesamtmasse des Materials üblicherweise zwischen 0,1 und 95 %, bevorzugt 1 bis 75 % und besonders bevorgzugt 5 bis 55 %. Der Füllstoffanteil beträgt in Abhängigkeit von der verwendeten Grundsubstanz 1 bis 98 %, bevorzugt bei 15 bis 95 % und besonders bevorzugt bei 40 bis 95 %. Der Katalysatoranteil, sofern von der Grundsubstanz her erforderlich, liegt zwischen 0,1 und 55 %, bevorzugt zwischen 1 bis 50 % und besonders bevorzugt zwischen 1,5 und 40 %.

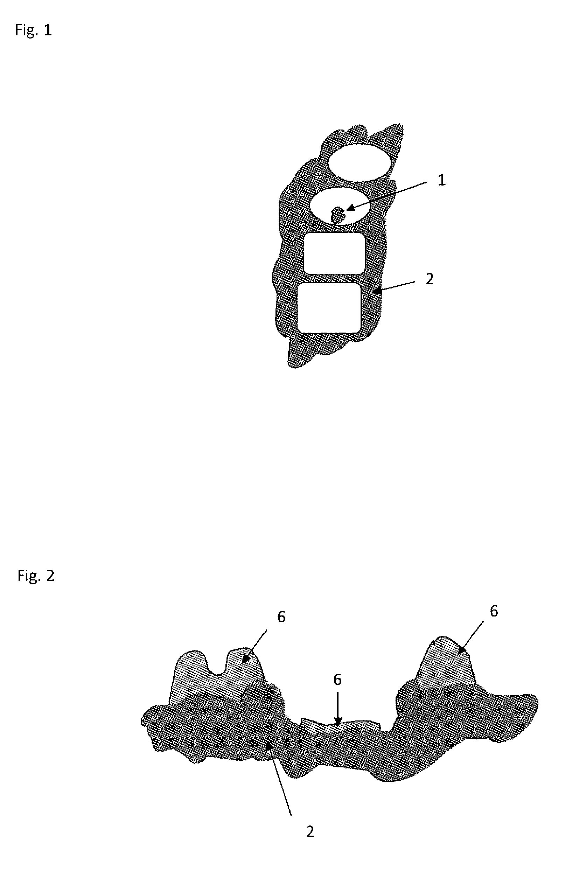

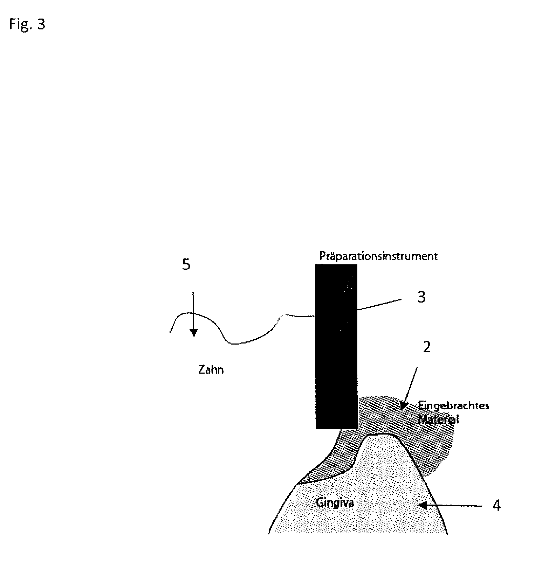

Nachfolgend wird die Erfindung anhand von Anwendungsbeispielen und den Figuren 1 bis 3 näher beschrieben:
1. Eine kariöse Läsion 1 soll mit Adhäsivtechnik und einer Kompositrestauration versorgt werden. Eine Kontamination mit Speichel soll verhindert werden. Dazu kann das erfindungsgemäße Material 2 großzügig um den relevanten Bereich verteilt werden, um das Operationsgebiet vor Blut, Speichel, Sulkusfluid zu schützen. Das Verfahren ist für alle Füllungsklassen anwendbar. Figur 1 zeigt diesen Anwendungsfall. Mit 2 ist das erfindungsgemäße Material bezeichnet.
2. Ein Zahn/mehrere Zähne soll/en endodontologisch versorgt werden. Auch hierbei übernimmt das Material Schutzfunktion. Hierbei wird auch das Gewebe des Patienten vor den Spülflüssigkeiten geschützt. Mit dem Material kann beispielsweise eine tulpenförmige Schutzvorrichtung "modelliert" werden, die den Patienten vor Zutritt reizender Spüllösungen in die Mundhöhle schützt und gleichzeitig eine Kontamination der Trepanationsöffnung mit Speichel o.ä. verhindert.
3. Ein Zahn/mehrere Zähne 6, 6' und 6" soll mit einem Stift/Stumpf- Aufbau versorgt werden, hierbei erfüllt das Material 2 die Trockenlegungsfunktion und anschließende Ausgangssituation für die intraorale Digitalisierung.
   (siehe Figur 2)
4. Ein Zahn 5 soll unter Verdrängung des Weichgewebes präpariert werden. Hierzu wird zuerst das Material 2 in den Sulkus appliziert, Ein Schutz vor Feuchtigkeit, Blut, etc. ist gewährleistet. Anschließend kann die Präparation mit einem Instrument 3 für eine geplante Restauration, egal ob prothetisch, konservierend oder Befestigungen für kieferorthopädische Apparaturen (z.B. Brackets) gewährleistet werden. Die Gingiva ist mit 4 bezeichnet.
   Das Material ist dabei vollständig in den Arbeitsschritt integrierbar. Es lässt sich durchbohren oder in Anteilen in die Präparation integrieren. So entsteht am Präparationsrand ein visuell deutlich erkennbarer Übergang(scharfe Grenze), der auch von Intraoralscannern deutlich erfasst werden kann. (siehe Figur 3)
5. Ein Zahn/mehrere Zähne soll mit einer intraoralen Digitalisierung abgeformt werden. Eine Anwendung des Materials direkt als Abformmaterial ermöglicht außerdem die extraorale Digitalisierung.
6. Ein Zahn soll abgeformt werden, das Material verbleibt im Mund und wird danach entfernt
7. Ein Zahn soll abgeformt werden und eine Modifikation des Materials soll sich während der Abformung mit der Abformmasse verbinden. Hierzu sind mehrere Varianten vorstellbar:
   a) Das Material verbindet sich chemisch trotz unterschiedlicher Phasen der Aushärtung
   b) Das Material muss mit einem adhäsiv und/oder Pulver mit retentiven Eigenschaften oder kleinen Perlen beschichtet werden.
   c) Das Material verbindet sich über Adhäsion
8. Das Material kann auch für die implantologische Abformung verwendet werden.
9. Das Material kann zum Schutz vor Feuchtigkeit bei der Befestigung von Brackets verwendet werden.
10. Das Material kann zum Schutz vor Feuchtigkeit bei der Befestigung von Adhäsivkonstruktionen (z.B. Adhäsivbrücken) verwendet werden.
11. Das Material kann zur temporären Befestigung von herausnehmbaren Apparaturen genutzt werden. Beispielsweise zur Verbesserung des Haltes einer DVT-Schablone. Durch seine selbsthärtende Eigenschaft entstehen Retentionen im Approximalbereich und in Unterschnitten.
12. Eine Kombination mit zahntechnischen Laborschritten und Abformmaterialien ist vorstellbar.
   a) Um eine großflächige Isolation der Mundhöhle, ähnlich der eines Kofferdams zu erreichen, könnte nach konventioneller Abformung und Gipsmodellherstellung im Labor die Herstellung einer Tiefziehschiene erfolgen. Am Patienten wird diese gegen das Material isoliert und anschließend mit dem Material bestückt. Nach Eingliederung während der flüssigen Phase und Aushärtung des Materials wird die Schiene entfernt, während das Material im Mund verbleibt und den jeweiligen Kiefer großflächig isoliert. Dies ist auch mit Teilsegmenten, Einzelzähnen etc. vorstellbar.
   b) Zur Erreichung des in a) genannten Zieles, ist auch eine Abformung am Patienten vorstellbar, die Bestückung der Abformung mit dem Material und die Reposition im Mund.
13. CAD/CAM: Da das Material für intra-/und extraorale Scanner erfassbar bzw. nicht erfassbar ist, sind Methoden zur Bissnahme oder Referenzierung mit dem Material vorstellbar.

## Patentansprüche

1. Dentalmaterial zum Management des paradontalen und periimplantären Weichgewebes, **dadurch gekennzeichnet, dass** das Material ein selbsthärtendes, mechanisch bearbeitbares Material ist, mit der Maßgabe, dass das ausgehärtete Material eine Shore A-Härte > 20 aufweist.

2. Dentalmaterial nach Anspruch 1, **dadurch gekennzeichnet, dass** das Material eine Shore A-Härte von bis zu 98 und eine Shore D-Härte von mindestens 20 bis 65 aufweist.

3. Dentalmaterial nach Anspruch 1 oder 2, **dadurch gekennzeichnet, dass** es digitalisierbar ist.

4. Dentalmaterial nach mindestens einem der Ansprüche 1 bis 3, **dadurch gekennzeichnet, dass** das Material fräsbar, schabbar, schleifbar und/oder schneidbar ist.

5. Dentalmaterial nach mindestens einem der Ansprüche 1 bis 4, **dadurch gekennzeichnet, dass** das Material ausgewählt ist aus Polysulfiden, alkalisch gelösten Acrylharzen, Kompositen oder dentalen Zementen.

6. Dentalmaterial nach mindestens einem der Ansprüche 1 bis 5, **dadurch gekennzeichnet, dass** das Material als Additive Füllstoffe, Absorber, Initiatoren, Farbstoffe, Adstringentien, Desinfektionsmittel, Vasokonstrikoren, Lösungsmittel und/oder Desentisizer enthält.

7. Dentalmaterial nach mindestens einem der Ansprüche 1 bis 6, **dadurch gekennzeichnet, dass** es vor der Aushärtung eine Viskosität von mindestens 500 mPa˙s bei 23°C, bevorzugt zwischen 10.00 mPa˙s und 100.000 mPa˙s aufweist.

8. Verfahren zum Management des paradontalen und periimplantären Weichgewebes, **dadurch gekennzeichnet, dass** ein Dentalmaterial nach mindestens einem der Ansprüche 1 bis 7 eingesetzt wird.

9. Verfahren nach Anspruch 7, **dadurch gekennzeichnet, dass** das Material appliziert und während der Abformung/Digitalisierung nicht entfernt wird.

10. Verfahren nach Anspruch 8, **dadurch gekennzeichnet, dass** das Material als Retraktionsmaterial eingesetzt wird und vor der Abformung/Digitalisierung entfernt wird.

11. Verwendung eines Dentalmaterials nach mindestens einem der Ansprüche 1 bis 8 zum Management des paradontalen und periimplantären Weichgewebes während der Präparation eines Zahns, der Abformung/Digitalisierung, der intraoralen Anfertigung einer Restauration, der Eingliederung einer extraoralen angefertigten Restauration.
